# EUROPEAN PATENT APPLICATION

(11) **EP 3 998 340 A1**
(43) Date of publication of application: **18.05.2022**
(21) Application number: 20836425.7
(22) Date of filing: 09.07.2020
(51) Int. Cl.: C12N 15/115, A61K 31/7088, A61P 35/00, G01N 33/574, G01N 33/53

(54) **APTAMER SPECIFICALLY BINDING TO KRAS PROTEIN AND METHOD FOR USING SAME**

(30) Priority: 10.07.2019 KR 20190083440
(71) Applicant: NUCLIXBIO, Guro-gu Seoul 08377 (KR)
(72) Inventor: KANG, Ho Young, Seoul 06624 (KR); KIM, Do Hyung, Gwangmyeong-si, Gyeonggi-do 14346 (KR); PARK, Kyoung Ryoung, Seoul 08335 (KR); BAEK, Seol Hwa, Seoul 07648 (KR)
(74) Representative: EIP
(86) International application number: PCT/KR2020/009032
(87) International publication number: WO 2021/006668

(57) **Abstract**

Provided are an aptamer specifically binding to KRAS protein, and a method of using the same. An aptamer of one aspect has excellent binding affinity to KRAS, which is a major factor regulating a cell growth signaling system *in vivo,* to inhibit binding between KRAS mutant in cancer cells and a Raf-1 kinase protein which is a downstream protein constituting the cell growth signaling system, thereby effectively suppressing cancer cell growth and cancer development. Further, the aptamer may specifically bind to KRAS in cancer cells, and thus may be widely applied to a pharmaceutical composition for treating or preventing cancer, a diagnostic agent, a reagent, etc.

## Description

### TECHNICAL FIELD

The present disclosure relates to an aptamer specifically binding to KRAS protein and a method of using the same.

### BACKGROUND ART

Aptamers, which are single-stranded DNA or RNA molecules having a structure such as stem, loop, hairpin, etc., are substances having an ability to bind to a specific protein, virus, peptide, etc. by adopting their own tertiary structures. Development of an aptamer that binds with high affinity to a specific target substance requires an *in vitro* process of screening for a small number of DNAs specifically binding to the target substance from a pool of randomly synthesized DNA libraries by repeating a process of allowing binding and separating between more than 1015 DNA libraries and the target substance. This method is called systematic evolution of ligands by exponential enrichment (SELEX).

Aptamers are known to have affinity high enough to be called substitutes for antibodies, and are more robust than antibodies which are proteins, because aptamers are DNA or RNA substances, can be mass-produced, and hardly cause immune rejection. Due to these characteristics, aptamers are more useful as therapeutic agents than existing protein drugs.

In addition, KRAS, which is one of major factors regulating a cell growth signaling system *in vivo,* is known as a representative oncogenic protein found in most cancer patients. In a normal state, KRAS regulates signal transduction by binding to GDP or GTP depending on generation of a cell growth signal, such as EGF, to exist in an inactivated or activated state. However, when a mutation occurs in a nucleotide sequence of the KRAS protein, in which the 12^{th} amino acid is changed from glycine to aspartate or valine, KRAS exists in a GTP-bound state at all times, and is abnormally activated, resulting in continuous downstream signaling of cell growth, leading to cancer.

In particular, since 30% to 50% of KRAS mutant proteins appear in colorectal cancer, lung cancer, etc., and 70% to 95% in pancreatic cancer, there is a problem in that it is still difficult to expect effective treatment results, when the KRAS mutant proteins are not under control even though anticancer agents are administered to cancer patients. The continuous cell growth signaling due to abnormal activation of KRAS mutant proteins indicates that KRAS mutation is a crucial factor in cancer progression along with the onset of cancer. Despite progress in the development of anticancer agents to control activated KRAS, a targeted anticancer agent that directly controls KRAS has not yet been developed, and accordingly, development thereof is needed.

### DESCRIPTION OF EMBODIMENTS

### TECHNICAL PROBLEM

An aspect provides an aptamer specifically binding to KRAS protein.

Another aspect provides a pharmaceutical composition for preventing or treating cancer, the pharmaceutical composition including the aptamer as an active ingredient.

Still another aspect provides a composition for detecting cancer cells or a kit for detecting cancer cells, each including the aptamer.

Still another aspect provides a method of providing information for diagnosing cancer by using the aptamer.

Still another aspect provides a method of preventing or treating cancer, the method including administering the aptamer to an individual in need thereof.

### SOLUTION TO PROBLEM

An aspect provides an aptamer which specifically binds to KRAS protein and includes a nucleotide sequence consisting of SEQ ID NO: 1 or SEQ ID NO: 2.

Hereinafter, the present disclosure will be described in more detail.

The "aptamer" is a single-stranded nucleic acid characterized by having a stable own tertiary structure while capable of binding to a target molecule with high affinity and specificity. The aptamer may be DNA, RNA, or a modified nucleic acid. Since a technology of developing aptamers, called systematic evolution of ligands by exponential enrichment (SELEX), was first developed, many aptamers capable of binding to various target molecules, including small-molecular organic materials, peptides, and membrane proteins, have been continuously developed. An aptamer is often compared to a single antibody due to its intrinsic characteristics of binding to a target molecule with high affinity and specificity, and is known to have a high potential as an alternative antibody, especially, referred to as a "chemical antibody."

KRAS protein is one of major factors regulating a cell growth signaling system *in vivo,* and is known as a representative oncogenic protein found in most cancer patients. In a normal state, KRAS regulates signal transduction by binding to GDP or GTP depending on generation of a cell growth signal, such as EGF, to exist in an inactivated or activated state. However, it is known that when a mutation occurs in an amino acid sequence of the KRAS protein to change the 12^{th} amino acid from glycine to aspartate or valine, KRAS exists in a GTP-bound state at all times, and is abnormally activated, resulting in continuous downstream signaling of cell growth, leading to cancer.

In addition to being prepared in the body of animals, KRAS proteins may be prepared using cells of mammals such as mice, etc., insect cells, cells such as E. *coli,* and may also be prepared by chemical synthesis. When KRAS proteins are prepared by cultured cells or chemical synthesis, variants may be easily prepared. Here, the variants refer to those having substitution of several amino acids or a partial amino acid sequence, and refer to proteins or peptides having at least one or more activities originally possessed by KRAS. When an amino acid is substituted, the substituted amino acid may be a natural amino acid or a non-natural amino acid. In one aspect, the KRAS protein particularly includes variants thereof, and for example, may be a mutant in which the 12^{th} amino acid in the amino acid sequence of the KRAS protein is changed from glycine to aspartate or valine.

The aptamer of one aspect specifically binds to a wild-type KRAS protein, specifically, KRAS^{G12D}, and inhibits an interaction between KRAS protein and Raf-1 protein to effectively inhibit cancer cell growth and oncogenic signaling activity, thereby exhibiting cancer cell-killing activity, which was demonstrated in a specific embodiment of the present disclosure.

The aptamer of an aspect specifically binds to KRAS protein or KRAS^{G12D} to inhibit binding of KRAS^{G12D} and Raf-1 kinase, thereby inhibiting signaling activity of KRAS derived from any mammal. The mammal may include, for example, primates (e.g., humans, monkeys), rodents (e.g., mice, rats, guinea pigs), and pets, livestock, and work animals (e.g., dogs, cats, horses, cattle, goats, sheep, pigs).

Inhibiting the signaling activity of KRAS means an ability to inhibit any signaling activity possessed by KRAS. For example, it is known that KRAS binds to Raf-1 kinase and then phosphorylates ERK through MEK, etc. to activate oncogenic signaling pathways. Through these signaling pathways, production of various cytokines or chemokines is induced. Accordingly, the KRAS signaling inhibitory activity may refer to an activity that inhibits production of cytokines, chemokines, etc., which exist downstream of the KRAS signaling pathway. Since expression of these cytokines or chemokines induces growth and development of cancer cells, the KRAS signaling inhibitory activity may also mean inhibition of their activity.

A length of the aptamer may be, not particularly limited, generally about 200 nucleotides or less. However, it is known that when the total number of nucleotides is small, chemical synthesis and mass production are easier, and it is also very advantageous in terms of cost. Therefore, from the viewpoint of application to pharmaceutical use, the aptamer of one aspect of the present disclosure may be, for example, about 10 nucleotides to about 200 nucleotides, about 50 nucleotides to about 150 nucleotides, about 55 nucleotides to about 100 nucleotides, and about 60 nucleotides to about 90 nucleotides in length.

The aptamer of an aspect may include a core sequence or a random sequence, which may be used interchangeably in the present disclosure. The core sequence means a core region capable of performing functions of the aptamer when it has a nucleotide sequence indicating the sequence thereof. The core sequence of the aptamer of an aspect may be any nucleotide sequence that may be used to increase the binding activity to KRAS, the inhibitory activity on binding of KRAS and Raf-1, etc., for example, a nucleotide sequence consisting of SEQ ID NO: 1 or 2. The aptamer of one aspect may refer to an aptamer having a form, in which the core sequence is combined with primer nucleotide sequences constantly conserved at both 5' and 3' ends (conserved primer region). The aptamer of one aspect may further include consecutive polynucleotides of 5 to 50 nucleotides at any one or more of the 5' end and the 3' end of the nucleotide consisting of SEQ ID NO: 1 or 2. The consecutive polynucleotides may be a primer sequence. The primer sequence of the aptamer means a sequence to which a primer may bind.

**[Table 1]**

| SEQ ID NO: | Sequence(5'-3') |
|---|---|
| 1 | ATC ATG NNC CGG GTT CGG NTG GGG GGN NNN NNC ATG ATC TAC |
| 2 | NCG GTA CGT CCG NNT ATG NGT GGG NGG TAA GGG AGA CCN C |
| 3 | NNN NNN NNN NNN GGN CAT NNG ATC ATG NNC CGG GTT CGG NTG GGG GGN NNN NNC ATG ATC TAC TTG ACT AGT ANA TGN CCN NNN NN |
| 4 | NNN NNN NNG ATC ATG NNC CGG GTT CGG NTG GGG GGN NNN NNC ATG ATC TAC TTG ACT AGT NNN |
| 5 | NNN NNN NNN NNN NNN CAT ANN NNN CGG TAC GTC CGN NTA TGN GTG GGN GGT AAG GGA GAC CNC NTG ACT AGT ACA NNN CCA CNN NN |
| 6 | NCA TAN NNN NCG GTA CGT CCG NNT ATG NGT GGG NGG TAA GGG AGA CCN CNT GAC TAG TAC ANN NCC ACN |

### (In the above general formula, each N is independently selected from A, T, G, and C, and those other than the underlined indicate the primer sequence.)

The aptamer of one aspect may be chemically synthesized by a method disclosed herein and by a method known per se in the art. An aptamer binds to a target substance in a wide variety of binding modes, such as ionic bonds based on a negative charge of a phosphate group, hydrophobic bonds and hydrogen bonds based on ribose, hydrogen bonds and stacking interaction based on nucleic acid bases, etc. In particular, ionic bonds based on the negative charge of the phosphate group, which are present in the same number as the number of constituent nucleotides, are strong, and bind to a positive charge of lysine or arginine being present on the surface of proteins. For this reason, nucleic acid nucleotides not involved in direct binding to a target substance may be substituted. In particular, because a region of a stem structure has already formed base pairs and faces the inside of a double helical structure, nucleic acid nucleotides are unlikely to directly bind to the target substance. Therefore, even when a nucleotide pair is substituted with another nucleotide pair, the activity of the aptamer often does not decrease. In structures wherein no nucleotide pairs are formed, such as loop structures, provided that the nucleic acid nucleotide is not involved in the direct binding to the target molecule, nucleotide substitution is possible.

An aptamer may be prepared by using a SELEX method or an improved version thereof (e.g., Ellington et al., (1990), Nature, 346, 818-822). In the SELEX method, by increasing the number of rounds or using a competing substance, an aptamer exhibiting a stronger binding potential for the target substance may be concentrated and selected. Therefore, by adjusting the number of rounds of SELEX and/or changing the competitive condition, aptamers with different binding forces, aptamers with different binding modes, and aptamers with the same binding force or binding mode but different nucleotide sequences may be obtained. The SELEX method also includes a process of amplification by PCR. By causing a mutation using manganese ions, etc. in the process, it is possible to perform SELEX with higher diversity.

The active aptamer thus selected may be subjected to optimized SELEX to obtain an aptamer having higher performance. In the optimized SELEX, SELEX may be performed again after preparing a template, wherein an aptamer with a determined sequence is partially randomized, or a template doped with about 10% to 30% of random sequences.

A ribose of a nucleotide included in the aptamer may be, for example, modified by substituting hydrogen at the C2'-position with a fluoro group or an amino group, or by substituting a hydroxy group (OH group) at the C2'-position by a methoxy group.

The aptamer may be any one of nucleotide sequences consisting of SEQ ID NOs: 3 to 6 shown below, or a conjugate of a plurality of the corresponding aptamers, as long as it binds to KRAS to inhibit binding of KRAS to Raf-1.

In the conjugate of a plurality of such aptamers, conjugation may be achieved by tandem binding. Further, in the conjugation, a linker may be used. As the linker, nucleotide chains (e.g., 1 nucleotide to about 20 nucleotides) and non-nucleotide chains (e.g., -(CH2)n- linker, -(CH2CH2O)n- linker, hexaethylene glycol linker, TEG linker, peptide-containing linker, -S-S- bond-containing linker, -CONH- bond-containing linker, - OPO3- bond-containing linker) may be mentioned. The plurality as mentioned in the above-described plural conjugates is not particularly limited, as long as it is two or more, and the plurality may be, for example, 2, 3 or 4.

The aptamer may include two or more stem-loop structures, wherein the loop structure may consist of 4 to 10 nucleotides.

In addition, the aptamer may be bound or labeled at one or more of the 5' end and the 3' end with one or more fluorescent materials selected from the group consisting of a fluorescent dye, a semiconductor nanocrystal, a lanthanide chelate, and a green fluorescent protein. The "fluorescent material" includes a material capable of emitting light, that is, as a signaling chromophore, a material capable of absorbing energy from other fluorescent materials and conjugated polyelectrolytes, for example, fluorescent dyes, semiconductor nanocrystals, lanthanide chelates, and fluorescent proteins. Examples of the fluorescent dyes include fluorescein, 6-carboxyfluorescein (6-FAM), 6-carboxytetramethylrhodamine (TAMRA), Rhodamine, Texas Red, tetramethylrhodamine, carboxyrhodamine, carboxyrhodamine 6G, carboxy rhodol, carboxy rhodamine 110, Cascade Blue, Cascade Yellow, Coumarin, Cy2 (brand name), Cy3 (brand name), Cy3.5 (brand name), Cy5 (brand name), Cy5.5 (brand name), Cy-chrome, phycoerythrin, peridinin chlorophyll-a protein (PerCP), PerCP-Cy5.5, 6-carboxy-4',5'-dichloro-2',7'-dimethoxyfluorescein (JOE), NED, 5-(and-6)-carboxy-X-rhodamine (ROX), HEX, Lucifer Yellow, Marina Blue, Oregon Green 488, Oregon Green 500, Oregon Green 514, Alexa Fluor (brand name) 350, Alexa Fluor (brand name) 430, Alexa Fluor (brand name) 488, Alexa Fluor (brand name) 532, Alexa Fluor (brand name) 546, Alexa Fluor (brand name) 568, Alexa Fluor (brand name) 594, AlexaFluor (brand name) 633, Alexa Fluor (brand name) 647, Alexa Fluor (brand name) 660, Alexa Fluor (brand name) 680, 7-amino-4-methylcoumarin-3-acetic acid, BODIPY (brand name) FL, BODIPY (brand name) FL-Br 2, BODIPY (brand name) 530/550, BODIPY (brand name) 558/568, BODIPY (brand name) 564/570, BODIPY (brand name) 576/589, BODIPY (brand name) 581/591, BODIPY (brand name) 630/650, BODIPY (brand name) 650/665, BODIPY (brand name) R6G, BODIPY (brand name) TMR, BODIPY (brand name) TR, conjugates thereof, and mixtures thereof. Examples of the lanthanide chelates may include europium chelate, terbium chelate, and samarium chelate. Another aspect provides a pharmaceutical composition for preventing or treating cancer, the pharmaceutical composition including the aptamer as an active ingredient.

The terms "subject", "individual", and "patient" are used interchangeably herein to refer to a vertebrate, mammal, or human.

Mammals include, but are not limited to, murines, monkeys, humans, farm animals, sports animals, and pets. Tissues, cells, and their progeny of a biological entity obtained *in vivo* or cultured *in vitro* are also included. The term "therapeutic agent" or "pharmaceutical composition" refers to a molecule or compound that confers some beneficial effect when administered to a subject.

The beneficial effect may include enabling of diagnostic decisions; improvement of a disease, symptom, disorder, or pathological condition; reduction or prevention of incidence of a disease, symptom, disorder, or illness; and general response to a disease, symptom, disorder, or pathological condition.

As used herein, "treatment" and "treating", or "alleviating" and "improving" are used interchangeably with each other. These terms refer to a method of obtaining an advantageous or desired result, including, but not limited to, a therapeutic benefit and/or a prophylactic benefit. The therapeutic benefit refers to any therapeutically significant improvement of one or more diseases, disorders, or symptoms under treatment, or effects thereon. In the prophylactic benefit, the composition may be administered to an individual at risk of developing a specific disease, disorder, or symptom, or to an individual reporting one or more physiological symptoms of the disease, even though the disease, disorder, or symptom has not yet appeared.

The term "effective amount" or "therapeutically effective amount" refers to an amount of an agent sufficient to produce an advantageous or desired result. The therapeutically effective amount may vary depending on one or more of a subject and pathological condition to be treated, a subject's body weight and age, severity of the pathological condition, mode of administration, etc., which may be easily determined by those skilled in the art. Further, the term is applied to the capacity to provide an image for detection by any of the imaging methods described herein. The specific dosage may vary depending on one or more of a particular agent selected, a dosage regimen that follows, whether or not it is administered in combination with another compounds, time of administration, a tissue being imaged and a body delivery system carrying the same.

The term "cancer" refers to a group of diseases characterized by excessive cell growth that invades surrounding tissues when the normal balance of apoptosis is disrupted. The cancer may be, for example, one or more selected from the group consisting of carcinoma derived from epithelial cells, such as lung cancer, laryngeal cancer, stomach cancer, colon/rectal cancer, liver cancer, gallbladder cancer, pancreatic cancer, breast cancer, ovarian cancer, uterine cancer, cervical cancer, prostate cancer, kidney cancer, carcinoma derived from epithelial cells, such as skin cancer, etc., sarcoma derived from connective tissue cells, such as osteosarcoma, myosarcoma, liposarcoma, fibrosarcoma, etc., melanoma, blood cancer derived from hematopoietic cells, such as leukemia, lymphoma, multiple myeloma, etc., tumors arising from nerve tissue. Specifically, the cancer may be pancreatic cancer, coloron cancer, or lung cancer.

The pharmaceutical composition for preventing or treating cancer may be used after mixing the aptamer with a pharmaceutically acceptable carrier, i.e., saline, sterile water, Ringer's solution, buffered saline, cyclodextrin, a dextrose solution, a maltodextrin solution, glycerol, ethanol, liposome, and one or more thereof. As needed, other common additives, such as antioxidants, buffers, etc., may be further included. In addition, the pharmaceutical composition may be formulated into injectable preparations, such as aqueous solutions, suspensions, emulsions, etc., pills, capsules, granules, or tablets, by further adding diluents, dispersants, surfactants, binders, and/or lubricants. Moreover, the pharmaceutical composition may be formulated depending on each component according to an appropriate method known in the art or a method disclosed in a Remington's literature (Remington's Pharmaceutical Science, Mack Publishing Company, Easton PA). The pharmaceutical composition may be formulated into, but is not particularly limited to, injectable or inhalable preparations. A method of administering the pharmaceutical composition according to an aspect may be, but is not particularly limited to, parenteral or oral administration, such as intravenous, subcutaneous, or intraperitoneal administration, inhalation or topical application, depending on a desired method.

The range of dosage varies in the range depending on a patient's body weight, age, sex, health status, diet, administration time, administration method, excretion rate, severity of disease, etc. A daily dose refers to an amount of the therapeutic material according to one aspect which is sufficient to treat an ameliorated disease state when administered to an individual in need thereof. An effective amount of the therapeutic material may depend on a specific compound, condition of a disease and severity thereof, an individual in need of treatment, and may be determined by those skilled in the art. For non-limiting example, the amount of the composition according to one aspect administered to the human body may vary depending on a patient's age, body weight, sex, mode of administration, health condition, and severity of the disease. Based on an adult patient with the body weight of 70 kg, the aptamer may be included in an effective amount of 0.1 µg/mL to 1,000 µg/mL, for example, 0.1 µg/mL to 500 µg/mL, 0.1 µg/mL to 100 µg/mL, 0.1 µg/mL to 50 µg/mL, 0.1 µg/mL to 25 µg/mL, 1 µg/mL to 1,000 µg/mL, 1 µg/mL to 500 µg/mL, 1 µg/mL to 100 µg/mL, 1 µg/mL to 50 µg/mL, 1 µg/mL to 25 µg/mL, 5µg/mL to 1,000 µg/mL, 5 µg/mL to 500 µg/mL, 5 µg/mL to 100 µg/mL, 5 µg/mL to 50 µg/mL, 5 µg/mL to 25 µg/mL, 10µg/mL to 1,000 µg/mL, 10 µg/mL to 500 µg/mL, 10 µg/mL to 100 µg/mL, 10 µg/mL to 50 µg/mL, or 10 µg/mL to 25 µg/mL, 10 µg/mL to 350µg/mL, 12.5 µg/mL to 300 µg/mL, 25 µg/mL to 270 µg/mL, 50 µg/mL to 150 µg/mL, or 100 µg/mL to 200 µg/mL per composition.

The composition may further include an anticancer agent, for example, a targeted anticancer agent. The targeted anticancer agent may refer to an anticancer agent that selectively kills only cancer cells by targeting a specific protein or a specific gene change that frequently occurs only in specific cancer cells and by blocking signals involved in cancer growth and development.

Still another aspect provides a composition for detecting cancer cells and a kit for detecting cancer cells, each including the aptamer.

The "detecting" simply refers to both "detecting" the presence of a target substance in a sample and "quantitative analysis" of the target substance in the sample.

The aptamer of one aspect may be bound to a fluorescent material for molecular imaging in order to diagnose cancer through imaging, and the detection agent may include the above substances.

The cancer cell detection agent may be the aptamer used as a detection probe, particularly, as a detection probe for KRAS variants. The method of labeling the aptamer is not particularly limited, and methods known per se are applicable. Such a method may include, for example, labeling with a radioisotope, labeling with a fluorescent pigment or dye, or a fluorescent protein.

The fluorescent material for molecular imaging refers to any material that generates fluorescence, for example, a material emitting red or near-infrared fluorescence, and a fluorescent material having a high quantum yield, but is not limited thereto.

The fluorescent material for molecular imaging may be a fluorescent material specifically biding to the aptamer, for example, a fluorescent protein or other imaging material, but is not limited thereto.

The fluorescent material may be, for example, fluorescein, BODIPY, tetramethylrhodamine, Alexa, cyanine, allophycocyanin, or a derivative thereof, but is not limited thereto.

The fluorescent protein may be, for example, Dronpa protein, green fluorescent protein (EGFP), red fluorescent protein (DsRFP), Cys3 or Cy5.5 which is a cyanine fluorescent material emitting near-infrared fluorescence, or other fluorescent protein, but is not limited thereto.

Other imaging materials may be, for example, iron oxide or a radioactive isotope, etc., but are not limited thereto, and may be applied to imaging equipment such as MRI and PET.

The kit may be a solid phase carrier, for example, a substrate, a resin, a plate (e.g., a multi-well plate), a filter, a cartridge, a column, and a porous material. The substrate may be those used in DNA chips, protein chips, etc., for example, nickel-polytetrafluoroethylene (PTFE) substrates, glass substrates, apatite substrates, silicon substrates, alumina substrates, etc. Substrates prepared by coating these substrates with a polymer, etc. may be mentioned. The resin may include, for example, agarose particles, silica particles, a copolymer of acrylamide and N,N'-methylenebisacrylamide, polystyrene crosslinked divinylbenzene particles, particles obtained by crosslinking dextran with epichlorohydrin, cellulose fiber, a crosslinked polymer of allyldextran and N,N'-methylenebisacrylamide, a monodisperse synthetic polymer, a monodisperse hydrophilic polymer, sepharose, Toyopearl, etc. Resins having a functional group bound thereto are also included. The kit may be useful, for example, for detection and quantification of KRAS protein. In addition, the kit includes, but is not limited to, an RT PCR kit, a micro array chip kit, a DNA kit, or a protein chip kit.

The aptamer may be immobilized on the kit by a method known per se. The method may be, for example, a method of introducing an affinity substance (e.g., those described above) or a predetermined functional group into the aptamer, followed by immobilization on a solid carrier via the affinity substance or the predetermined functional group. The predetermined functional group may be a functional group that may be subjected to a coupling reaction, for example, an amino group, a thiol group, a hydroxyl group, and a carboxyl group. The present disclosure also provides an aptamer into which such a functional group is introduced.

The kit of one aspect may include the aptamer, etc. for the detection of cancer cells, as well as one or more kinds of other component compositions, solutions, or devices suitable for the analysis method.

In addition, the kit for detecting cancer cells of one aspect may include a substrate, an appropriate buffer solution, a secondary antibody labeled with a chromogenic enzyme or fluorescent material, and a chromogenic substrate for the detection of cancer cells. Herein, as the substrate, a nitrocellulose membrane, a 96-well plate synthesized from a polyvinyl resin, a 96-well plate synthesized from a polystyrene resin, and a glass slide glass may be used. As the chromogenic enzyme, peroxidase, alkaline phosphatase, etc. may be used. As the fluorescent material, FITC, RITC, etc., may be used. As the color development substrate solution, 2,2'-azino-bis(3-ethylbenzothiazoline-6-sulfonic acid), O-phenylenediamine (OPD), or tetramethylbenzidine (TMB) may be used.

Still another aspect provides a method of providing information for diagnosing cancer, the method including bringing a biological sample into contact with the aptamer to form an aptamer-KRAS complex; measuring a level of the aptamer-KRAS complex; and comparing the measured level of the aptamer-KRAS complex with a level of a control group and diagnosing as cancer when the measured level is higher than that of the control.

The biological sample refers to a sample separated from an individual, specifically, selected from blood, serum, plasma, saliva, urine, and tissue samples, but is not limited thereto.

The method may include determining that an individual has cancer when a measured aptamer-KRAS complex level of the individual is higher than an aptamer-KRAS complex level of a control sample without cancer. Further, in the method, the control refers to a sample including no cancer cells, which is separated from a normal person without cancer.

The method of providing information for diagnosing cancer may include detecting KRAS^{G12D}-activated cancer cells and measuring the detection level thereof, instead of measuring the aptamer-KRAS complex level, by detecting KRAS^{G12D}, which is a KRAS mutant expressed in cancer cells and is activated in cancer cell growth and oncogenic signaling. In this case, in the determining cancer, the individual may be determined as having cancer when the measured KRAS^{G12D} activation level is higher than an average detection level of a sample of a control which is an individual without cancer. The methods of detecting the measured cancer cells and detecting and quantifying the activated KRAS^{G12D} may be performed in the same manner as an immunological method, except that the aptamer of the present disclosure is used in place of an antibody. Therefore, by using the aptamer of the present disclosure in place of an antibody, in the same manner as in methods such as enzyme immunoassay (EIA) (e.g., direct competitive ELISA, indirect competitive ELISA, sandwich ELISA), radioimmunoassay (RIA), fluorescent immunoassay (FIA), a Western blot method (e.g., used instead of a secondary antibody in the Western blot method), an immunohistochemical staining method, and a cell sorting method, etc., detection and quantitation may be performed. These methods may be useful not only in, for example, measuring the KRAS mutant level in living organisms or biological samples, or diagnosing a disease associated with KRAS signaling, but also for objects other than disease diagnosis such as scientific object, experiment and study object, etc., including detection or quantification of KRAS by using the aptamer of the present disclosure instead of an antibody, and a biological sample derived from human or animal other than human, or a sample other than biological samples.

Still another aspect provides a method of preventing or treating cancer, the method including administering the aptamer to an individual in need thereof.

The redundant contents are omitted in consideration of complexity of the present specification, and the terms not otherwise defined in the present specification have the meanings commonly used in the technical field to which the present disclosure pertains.

### ADVANTAGEOUS EFFECTS OF DISCLOSURE

An aptamer of one aspect has excellent binding affinity to KRAS, which is a major factor regulating a cell growth signaling system *in vivo,* to inhibit binding between KRAS mutant in cancer cells and a Raf-1 kinase protein which is a downstream protein constituting the cell growth signaling system, thereby effectively suppressing cancer cell growth and cancer development. Further, the aptamer may specifically bind to KRAS in cancer cells, and thus may be widely applied to a pharmaceutical composition for treating or preventing cancer, a diagnostic agent, a reagent, etc.

### BRIEF DESCRIPTION OF DRAWINGS

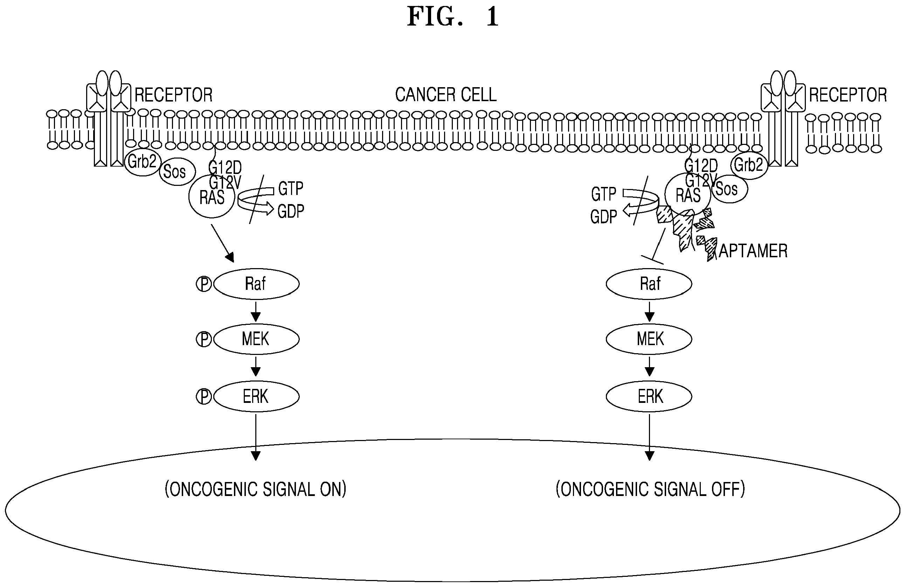

FIG. 1 is an illustration showing that a KRAS protein-specific aptamer is able to regulate a tumor growth signaling system induced by interaction between KRAS and Raf-1;
FIGS. 2A, 2B, and 2C show results of ELISA experiments to examine binding affinity between KRAS^{G12D} protein and selected aptamers, respectively;
FIGS. 3A, 3B, 3C, and 3D show secondary structures predicted from nucleotide sequences of four finally selected aptamers, NBK1 (FIG. 3A), NBK1-1 (FIG. 3B), NBK2 (FIG. 3C), and NBK2-1 (FIG. 3D);
FIG. 4 shows results of ELISA experiments showing that binding between KRAS^{G12D} and Raf-1 protein may be inhibited by the selected four kinds of aptamers; and
FIG. 5 shows images (FIG. 5, top) of fluorescence detection of actual binding affinity between KRAS protein and NBK1 and NBK2 aptamers transfected into a pancreatic cancer cell line BxPC-3, and results of measuring cell viability using a CellTiter-Glo (FIG. 5, bottom).

### MODE OF DISCLOSURE

Hereinafter, an aspect will be described in more detail with reference to embodiments and experimental embodiments. However, these embodiments and experimental embodiments are only for illustrating an aspect, and the scope of an aspect is not limited to these embodiments and experimental embodiments. Embodiments and experimental embodiments of an aspect are provided to more fully explain an aspect to those skilled in the art.

Example 1. Mass Production and Isolation of KRAS Protein and Raf-1 (RAS binding domain) Protein

For KRAS expression, a pReceiver-B01 (Genecopoeia) gene having 6-histidine tags at the N-terminus and encoding a wild-type KRAS gene was purchased. To prepare a vector for KRAS^{G12D} mutant protein expression, the gene was amplified by polymerase chain reaction (PCR) using a forward primer (F: GTAGTTGGAGCTGATGGCGTAGGCAAG) and a reverse primer (R: CTTGCCTACGCCATCAGCTCCAACTAC). As a result, 6-histidine-tagged human KRAS wild-type loaded with GDP and KRAS^{G12D} mutant protein loaded with GMPPNP were obtained. For protein expression, transformation into *E.coli* Rosetta DE3 was performed, followed by incubation using luria-bertani (LB) at 37°C until OD₆₀₀ reached about 0.6. Thereafter, to induce protein expression, 0.1 mM β-D-1-thiogalactopyranoside (IPTG) was added. Incubation was performed at 37°C for 2 hours and 30 minutes, and then a cell pellet was recovered. The recovered *E.coli* cells were disrupted with a sonicator using a lysis buffer containing 50 mM tris-HCI (pH 7.5), 500 mM NaCl, 0.1 % triton X-100, 14.3 mM β-mercaptoethanol, 1 mM PMSF, and 10% glycerol, and a soluble fraction was obtained by centrifugation. Affinity chromatography was performed using a HisTrap HP column. Thereafter, dialysis was performed using a desalting column, and additional purification was performed using an ion-exchange chromatography column.

Example 2. Preparation of KRAS Wild-type and KRAS^{G12D} (GMPPNP loaded form) Protein

Using the protein purified in Example 1, KRAS wild-type protein was dialyzed against a GDP exchange buffer containing 50 mM Tris-HCI (pH 8), 1 mM DTT, and 2 mM MgCl₂, followed by adding 45 times GDP and about 20 mM EDTA. Incubation was performed at 30°C for 30 minutes, and 50 mM MgCl₂ was further added, followed by incubation at 4°C for 30 minutes. Then, the protein was dialyzed against a buffer solution containing 25 mM Tris-HCI (pH 7.5), 100 mM NaCl, 2 mM MgCl₂, 1 mM DTT, and 10% glycerol. KRAS^{G12D} protein was dialyzed against an exchange buffer containing 40 mM Tris-HCI (pH 7.5), 200 mM (NH₄)₂SO₄, 10 µM ZnCl₂, and 5 mM DTT GMPPNP. 45 times GMPPNP and alkaline phosphate beads were added, followed by incubation at 25 °C for 90 minutes. Thereafter, the beads were removed and then dialyzed against the same buffer as used for the KRAS wild-type protein.

Example 3. Selection of Aptamer Specifically Binding to KRAS and Identification of Structure Thereof

An experiment was performed to effectively select an aptamer that specifically binds only to KRAS protein. A single-stranded DNA aptamer capable of specifically binding to a KRAS mutant protein (KRAS^{G12D}) was selected from random libraries consisting of 8×10¹⁴ DNA molecules through Systemic Evolution of Ligands by Exponential Enrichment (SELEX) technique. At this time, single-stranded DNA libraries (Trilink Biotechnologies USA) consisting of 23 consecutive primer sequences at the 5' and 3' ends of 40 random sequences were purchased and used, and a necessary template DNA was amplified through PCR. Thereafter, an experiment was performed to select a KRAS-specific aptamer from the products amplified by the PCR by varying the KRAS concentration, and experimental conditions for each round are shown in Table 2 below.

**[Table 2]**

| Number of round | 1 | 2 | 3 | 4 | 5 | 6 | 7 | 8 | 9 | 10 |
|---|---|---|---|---|---|---|---|---|---|---|
| KRAS concentration (pmol) | 425.5 | | 255.3 | | | | 128.0 | | | |
| ssDNA concentration (pmol) | 1276.6 | | | | | | 1282.1 | | | |
| Buffer composition | 50 mM tris-HCI (pH 7.5), 150 mM NaCl, 1 mM MgCl₂, 0.4 mM PMSF, 5% glycerol, and 14.3 mM β-mercaptoethanol | | | | | | | | | |

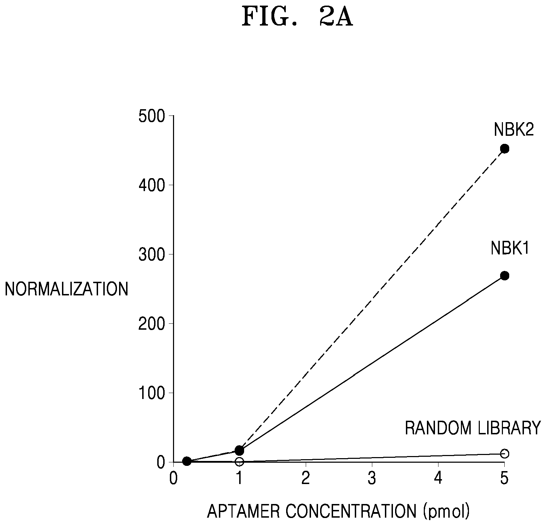

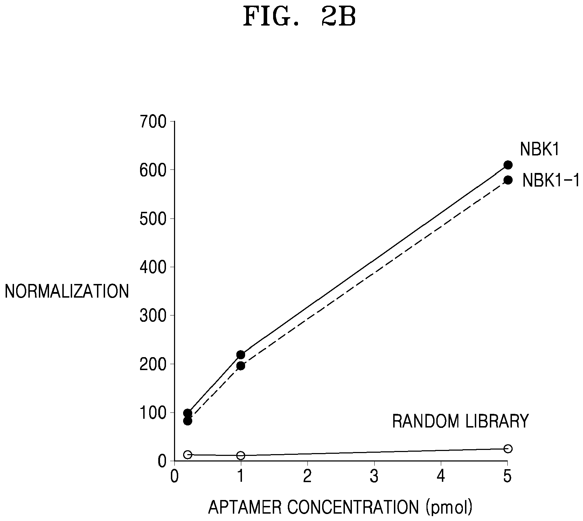

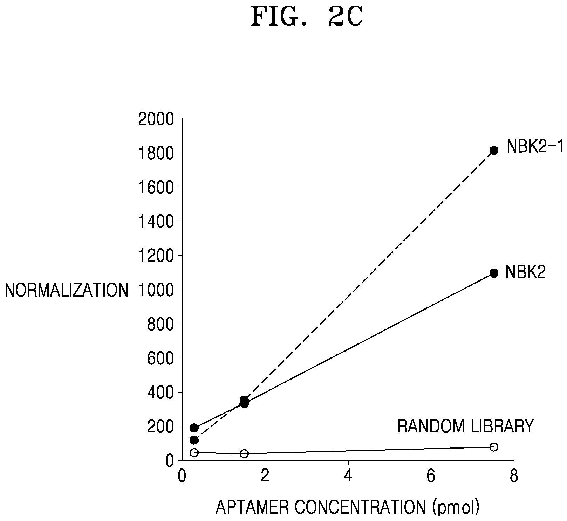

The selection and PCR amplification processes were repeated 10 times using the above conditions, and a total of 4 types of aptamers, including 2 types showing the highest binding affinity to the KRAS mutant protein (KRAS^{G12D}) in the libraries and 2 types obtained by trimming each aptamer, were subjected to an ELISA experiment to determine the binding affinity, and the results thereof are shown in FIG. 2.

As in FIG. 2, it was confirmed that all the four aptamer candidates finally selected exhibited a significant binding affinity to KRAS^{G12D} protein when compared with the sequences of the random libraries, and the binding affinity increased in an aptamer concentration-dependent manner. In particular, the binding affinity between the KRAS mutant protein and NBK2-1, 2 was found to be remarkably high. As described above, the final four aptamer sequences capable of binding to KRAS with high affinity were identified, and shown in Table 3 below.

**[Table 3]**

| Aptamer ID | Nucleotides sequence | Sequence length |
|---|---|---|
| NBK1 | 5'- NNN NNN NNN NNN GGN CAT NNG ATC ATG NNC CGG GTT CGG NTG GGG GGN NNN NNC ATG ATC TAC TTG ACT AGT ANA TGN CCN NNN NN -3' | 86 mer |
| NBK1-1 | 5'- NNN NNN NNG ATC ATG NNC CGG GTT CGG NTG GGG GGN NNN NNC ATG ATC TAC TTG ACT AGT NNN-3' | 63 mer |
| NBK2 | 5'-NNN NNN NNN NNN NNN CAT ANN NNN CGG TAC GTC CGN NTA TGN GTG GGN GGT AAG GGA GAC CNC NTG ACT AGT ACA NNN CCA CNN NN -3' | 86 mer |
| NBK2-1 | 5'-NCA TAN NNN NCG GTA CGT CCG NNT ATG NGT GGG NGG TAA GGG AGA CCN CNT GAC TAG TAC ANN NCC ACN-3' | 69 mer |

(In the above general formula, each N is independently selected from A, T, G, and C, and those other than the underlined indicate the primer sequence.)

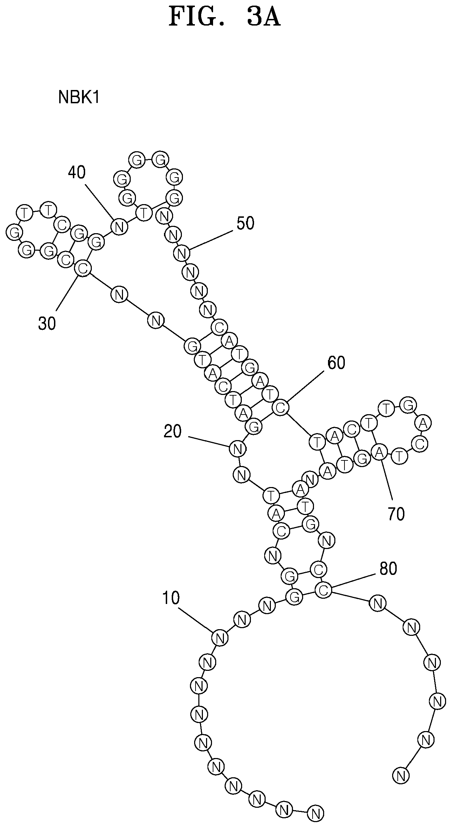

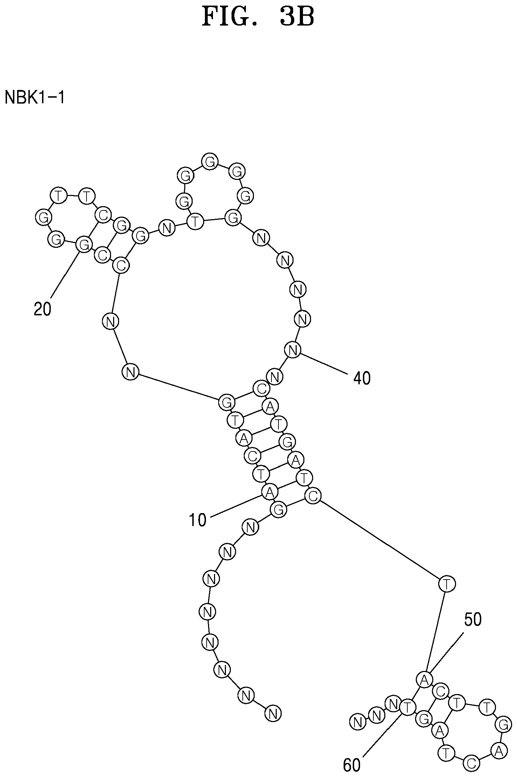

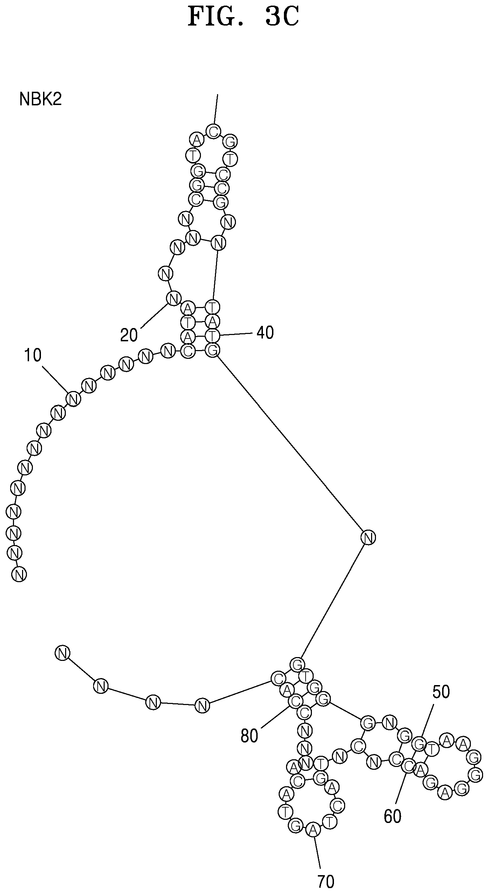

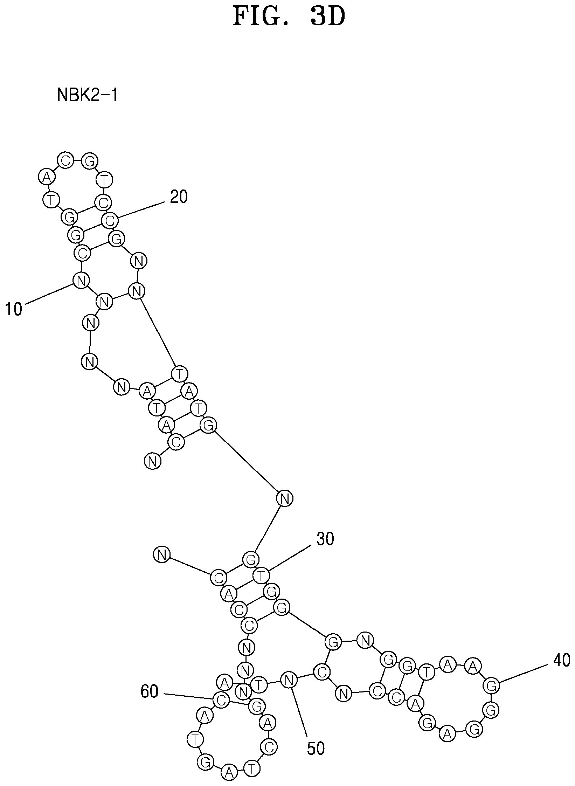

Secondary structures of the selected DNA aptamers represented by the above sequences were predicted through RNA structures, and the structures thereof are shown in FIGS. 3A, 3B, 3C, and 3D, respectively.

As shown in FIGS. 3A, 3B, 3C, and 3D, it was confirmed that the KRAS-specific aptamers finally selected had four different types of secondary structures.

Example 4. Examination of Effect of Biological Anticancer Activity Inhibition of KRAS-Specific Binding Aptamer

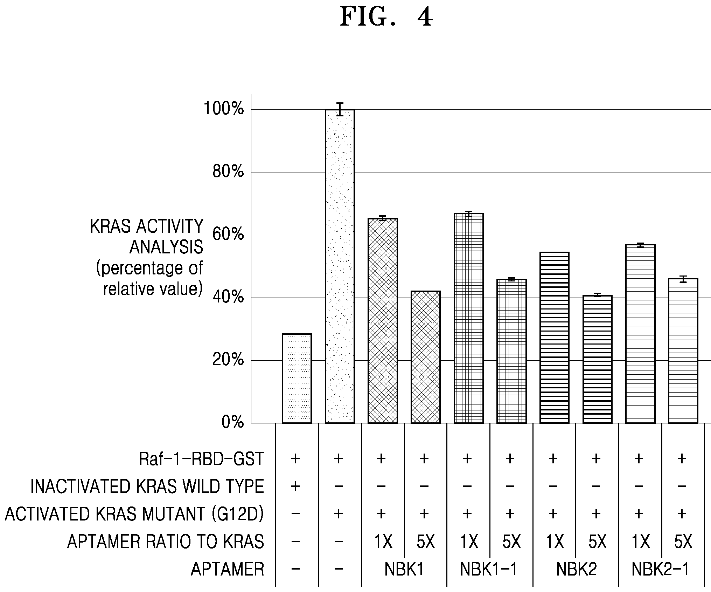

To examine whether the four kinds of aptamers selected in Example 3 interact with KRAS to competitively act with an oncogenic signal Raf-1, thereby inhibiting excessive proliferation signals of cancer cells and exhibiting anticancer activity, ELISA was performed. The four kinds of aptamers finally selected were treated at concentrations 1, 5 times the concentration of KRAS protein and allowed to react in PBS buffer at 37°C, followed by ELISA assay. In order to effectively identify the actual inhibitory reaction, GST tagged Raf-1 protein was treated with KRAS^{G12D} protein and DNA aptamer at the same time, a group treated with an inactivated wild-type KRAS protein was used as a negative control group, and a group treated with only an activated KRAS^{G12D} protein was used as a positive control group, which were determined as a percentage of 100%. The other groups were determined as a relative value thereto. Experiments were performed and results thereof are shown in FIG. 4.

As in FIG. 4, it was confirmed that, when the four kinds of aptamers finally selected were treated, they effectively bound to KRAS in a concentration-dependent manner, thereby effectively suppressing the oncogenic signal of Raf-1, as compared with the positive control group. In particular, it was confirmed that, when the four kinds of aptamers were treated at concentrations 5 times the KRAS protein concentration, the KRAS activity could be significantly inhibited to about 1/2 on average, as compared with the positive control group.

Example 5. Examination of Cancer Cell-Killing Effect of KRAS-specific binding aptamer *In vitro*

As in Example 4, it was confirmed that the aptamers finally selected inhibited the interaction between the purified KRAS proteins, and thus the KRAS activity-inhibiting ability of the aptamers was confirmed. An experiment was performed to investigate the efficacy of the aptamers against actual cancer cells. In detail, a BxPC-3 pancreatic cancer cell line, which is a KRAS-expressing cell, was treated with the aptamers to examine whether the aptamers actually exhibit the cancer cell-killing effects.

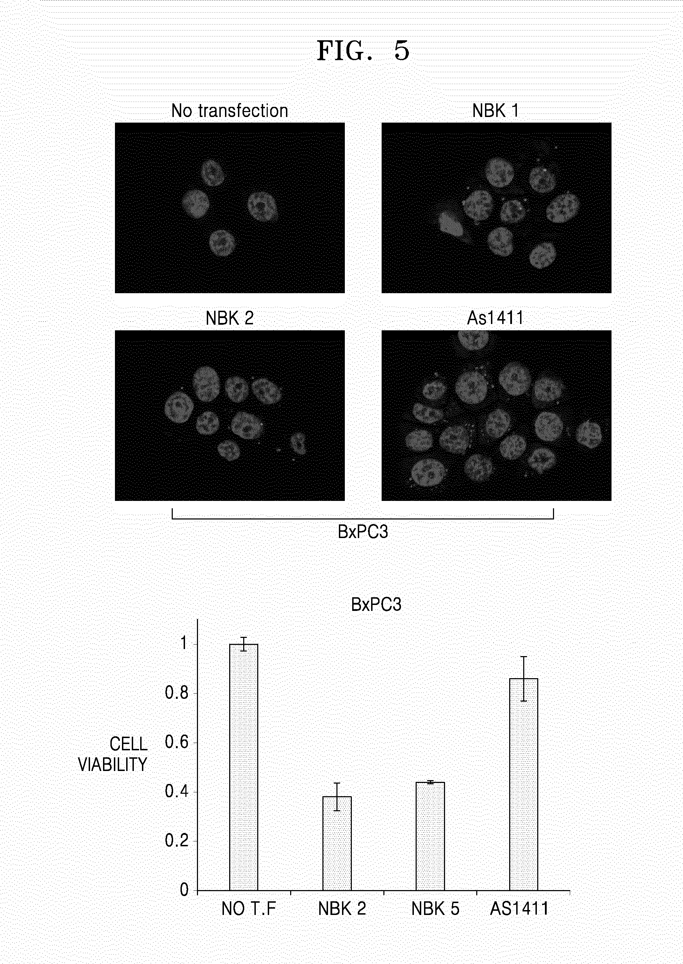

60 nM of Cy3-labeled NBK1 or NBK2 aptamer and AS1411 which is an aptamer having a cancer cell-targeting function were determined as control groups. To react each aptamer with BxPC-3 cells, cells were first seeded. After 24 hours, each aptamer was transfected into the cells using Lipofectamine 3000 reagent. After 24 hours, the cells were detached and then seeded again in a 96-well plate. On day 3 after transfection, the cells were examined through a fluorescence microscope. The results of examining the cells through the fluorescence microscope are shown in the top of FIG. 5, and the results of measuring viability of cancer cells by measuring cell viability using a CellTiter-Glo are shown in the bottom of FIG. 5.

As in the top and bottom of FIG. 5, it was confirmed that when the NBK1 and NBK2 aptamers finally selected were brought into contact with the BxPC-3 pancreatic cancer cell line, the viability of cancer cells was reduced by 1/2 or more. Even compared with the aptamer AS1411, the viability was confirmed to be reduced by about 50% or more. Taken together, it was confirmed that when the NBK1 and NBK2 aptamers finally selected are used, they specifically bind to KRAS in cancer cells to effectively inhibit tumorigenic signals, thereby significantly killing cancer cells.

## Claims

1. An aptamer which specifically binds to KRAS protein and comprises a nucleotide sequence consisting of SEQ ID NO: 1 or SEQ ID NO: 2.

2. The aptamer of claim 1, further comprising consecutive polynucleotides of 5 to 50 nucleotides at any one or more of the 5' end and the 3' end of the aptamer.

3. The aptamer of claim 2, wherein the consecutive polynucleotides are primer sequences.

4. The aptamer of claim 2, wherein the aptamer comprises any one of nucleotide sequences consisting of SEQ ID NOS: 3 to 6.

5. The aptamer of claim 2, wherein the aptamer comprises two or more stem-loop structures.

6. The aptamer of claim 5, wherein the loop structure consists of 4 to 10 nucleotides.

7. The aptamer of claim 1, wherein the aptamer is labeled at any one or more of the 5' end and the 3' end with one or more fluorescent materials selected from the group consisting of a fluorescent dye, a semiconductor nanocrystal, a lanthanide chelate, and a green fluorescent protein.

8. The aptamer of claim 1, wherein the aptamer inhibits binding between the KRAS protein and Raf-1 protein.

9. A pharmaceutical composition for preventing or treating cancer, the pharmaceutical composition comprising the aptamer of claim 1 as an active ingredient.

10. The pharmaceutical composition of claim 9, wherein the cancer is one or more selected from the group consisting of lung cancer, laryngeal cancer, stomach cancer, colon/rectal cancer, liver cancer, gallbladder cancer, pancreatic cancer, breast cancer, ovarian cancer, uterine cancer, cervical cancer, prostate cancer, kidney cancer, osteosarcoma, myosarcoma, liposarcoma, melanoma, leukemia, lymphoma, blood cancer, and tumors arising from nerve tissue.

11. A composition for detecting cancer cells, the composition comprising the aptamer of claim 1.

12. A kit for detecting cancer cells, the kit comprising the aptamer of claim 1.

13. A method of providing information for diagnosing cancer, the method comprising bringing a biological sample into contact with the aptamer of claim 1 to form an aptamer-KRAS complex;
measuring a level of the aptamer-KRAS complex; and
comparing the measured level of the aptamer-KRAS complex with a level of a control group and diagnosing as cancer when the measured level is higher than that of the control group.

14. A method of preventing or treating cancer, the method comprising administering the aptamer of claim 1 to an individual in need thereof.


<110>	Nuclixbio

<120>	Aptamer for KRAS and use thereof

<130>	PX062311

<150>	KR 10-2019-0083440
<151>	2019-07-10

<160>	8

<170>	KoPatentIn 3.0

<210>	1
<211>	42
<212>	DNA
<213>	Artificial Sequence

<220>
<223>	common sequence of aptamer sequence of NBK1 and NBK1-1


<220>
<221>	misc_feature
<222>	(7)..(8)
<223>	each n is A, T, G or C


<220>
<221>	misc_feature
<222>	(19)
<223>	each n is A, T, G or C


<220>
<221>	misc_feature
<222>	(27)..(32)
<223>	each n is A, T, G or C


<400>	1
atcatgnncc gggttcggnt ggggggnnnn nncatgatct ac	42


<210>	2
<211>	40
<212>	DNA
<213>	Artificial Sequence

<220>
<223>	common sequence of aptamer sequence of NBK2 and NBK2-1


<220>
<221>	misc_feature
<222>	(1)
<223>	each n is A, T, G or C


<220>
<221>	misc_feature
<222>	(13)..(14)
<223>	each n is A, T, G or C

<220>
<221>    misc_feature
<222>    (19)
<223>    each n is A, T, G or C


<220>
<221>    misc_feature
<222>    (25)
<223>    each n is A, T, G or C


<220>
<221>    misc_feature
<222>    (39)
<223>    each n is A, T, G or C


<400>    2
ncggtacgtc cgnntatgng tgggnggtaa gggagaccnc                                40


<210>    3
<211>    86
<212>    DNA
<213>    Artificial Sequence

<220>
<223>    aptamer sequence of NBK1


<220>
<221>    misc_feature
<222>    (1)..(12)
<223>    each n is A, T, G or C


<220>
<221>    misc_feature
<222>    (15)
<223>    each n is A, T, G or C


<220>
<221>    misc_feature
<222>    (19)..(20)
<223>    each n is A, T, G or C


<220>
<221>    misc_feature
<222>    (28)..(29)
<223>    each n is A, T, G or C


<220>
<221>    misc_feature
<222>    (40)
<223>    each n is A, T, G or C

<220>
<221>    misc_feature
<222>    (48)..(53)
<223>    each n is A, T, G or C


<220>
<221>    misc_feature
<222>    (74)
<223>    each n is A, T, G or C


<220>
<221>    misc_feature
<222>    (78)
<223>    each n is A, T, G or C


<220>
<221>    misc_feature
<222>    (81)..(86)
<223>    each n is A, T, G or C


<400>    3
nnnnnnnnnn nnggncatnn gatcatgnnc cgggttcggn tggggggnnn nnncatgatc    60

tacttgacta gtanatgncc nnnnnn                                        86


<210>    4
<211>    63
<212>    DNA
<213>    Artificial Sequence

<220>
<223>    aptamer sequence of NBK1-1


<220>
<221>    misc_feature
<222>    (1)..(8)
<223>    each n is A, T, G or C


<220>
<221>    misc_feature
<222>    (16)..(17)
<223>    each n is A, T, G or C


<220>
<221>    misc_feature
<222>    (28)
<223>    each n is A, T, G or C


<220>
<221>    misc_feature
<222>    (36)..(41)
<223>    each n is A, T, G or C

<220>
<221>    misc_feature
<222>    (61)..(63)
<223>    each n is A, T, G or C

<400>    4
nnnnnnnnga tcatgnnccg ggttcggntg gggggnnnnn ncatgatcta cttgactagt        60

nnn                                                                      63

<210>    5
<211>    86
<212>    DNA
<213>    Artificial Sequence

<220>
<223>    aptamer sequence of NBK2

<220>
<221>    misc_feature
<222>    (1)..(15)
<223>    each n is A, T, G or C

<220>
<221>    misc_feature
<222>    (20)..(24)
<223>    each n is A, T, G or C

<220>
<221>    misc_feature
<222>    (36)..(37)
<223>    each n is A, T, G or C

<220>
<221>    misc_feature
<222>    (42)
<223>    each n is A, T, G or C

<220>
<221>    misc_feature
<222>    (48)
<223>    each n is A, T, G or C

<220>
<221>    misc_feature
<222>    (62)
<223>    each n is A, T, G or C

<220>
<221>    misc_feature
<222>    (64)
<223>    each n is A, T, G or C

<220>
<221>    misc_feature
<222>    (76)..(78)
<223>    each n is A, T, G or C


<220>
<221>    misc_feature
<222>    (83)..(86)
<223>    each n is A, T, G or C


<400>    5
nnnnnnnnnn nnnnncatan nnnncggtac gtccgnntat gngtgggngg taagggagac        60

cncntgacta gtacannncc acnnnn                                              86


<210>    6
<211>    69
<212>    DNA
<213>    Artificial Sequence

<220>
<223>    aptamer sequence of NBK2-1


<220>
<221>    misc_feature
<222>    (1)
<223>    each n is A, T, G or C


<220>
<221>    misc_feature
<222>    (6)..(10)
<223>    each n is A, T, G or C


<220>
<221>    misc_feature
<222>    (22)..(23)
<223>    each n is A, T, G or C


<220>
<221>    misc_feature
<222>    (28)
<223>    each n is A, T, G or C


<220>
<221>    misc_feature
<222>    (34)
<223>    each n is A, T, G or C


<220>
<221>    misc_feature
<222>    (48)
<223>    each n is A, T, G or C

<220>
<221>    misc_feature
<222>    (50)
<223>    each n is A, T, G or C


<220>
<221>    misc_feature
<222>    (62)..(64)
<223>    each n is A, T, G or C


<220>
<221>    misc_feature
<222>    (69)
<223>    each n is A, T, G or C


<400>    6
ncatannnnn cggtacgtcc gnntatgngt gggnggtaag ggagaccncn tgactagtac        60

annnccacn                                                                69


<210>    7
<211>    27
<212>    DNA
<213>    Artificial Sequence

<220>
<223>    forward primer sequence


<400>    7
gtagttggag ctgatggcgt aggcaag                                            27


<210>    8
<211>    27
<212>    DNA
<213>    Artificial Sequence

<220>
<223>    reverse primer sequence


<400>    8
cttgcctacg ccatcagctc caactac                                            27